# EUROPEAN PATENT APPLICATION

(11) **EP 3 315 174 A1**
(43) Date of publication of application: **02.05.2018**
(21) Application number: 17199334.8
(22) Date of filing: 31.10.2017
(51) Int. Cl.: A61Q 19/00, A61K 8/92, A61K 8/97, A61K 36/185

(54) **SEED OIL, COMPOSITION COMPRISING SEED OIL AND USE OF SEED OIL**

(30) Priority: 01.11.2016 FI 20165823
(71) Applicant: Aromtech Ltd, 95410 Tornio (FI)
(72) Inventor: Määttä, Petri, 95440 Tornio (FI); Larmo, Petra, 95400 Tornio (FI); Kemppainen, Lauri, 95420 Tornio (FI); Sorsa, Pia, 94400 Keminmaa (FI)
(74) Representative: Berggren Oy, Turku

(57) **Abstract**

The present invention provides a seed oil, obtainable from seeds of *Hippophae rhamnoides* by supercritical CO₂ extraction, the seed oil comprising at least 25 weight-% linoleic acid calculated from the total fatty acids in the seed oil, at least 20 weight-% alpha-linolenic acid calculated from the total fatty acids in the seed oil, less than 0.001 weight-% carotenoids, based on the total weight of the seed oil, and wherein the amount of polycyclic aromatic hydrocarbons (PAHs), selected from the group consisting of benzo(a)pyrene, chrysene, benz(a)anthracene and benzo(a)fluoranthene is less than 10 µg/kg seed oil.

## Description

### TECHNICAL FIELD OF THE INVENTION

The present invention relates to a seed oil and a composition comprising a seed oil according to the preambles of the appended independent claims. The invention relates also to a use of a seed oil.

### BACKGROUND OF THE INVENTION

Sea buckthorn (*Hippophae rhamnoides*) berries have been used for centuries for food, therapeutic, and pharmaceutical purposes due to their nutritional profile. The berries contain hundreds of bioactive compounds, including vitamins A, C, E, F, K and B, carotenoids, flavonoids, biotin, folic acid, and various dietary minerals and trace minerals, as well as essential fatty acids. The bioactive components vary with fruit maturity, fruit size, species, geographic locations, climate and methods of extraction. The valuable part of the sea buckthorn is its oil which can be extracted from either the seeds or the pulp of the fruit.

The sea buckthorn seed oil provides a source for polyunsaturated fatty acids. The major fatty acids in the seed oil are omega 3 and 6, particularly linoleic acid and alpha-linolenic acid. Sea buckthorn seed oil contains also carotenoids and tocopherols. Additionally, it contains β-sitosterol, but no cholesterol or stigmasterol.

Traditionally, the sea buckthorn oil has been extracted from the berries by cold-pressing or extracting with an organic solvent. More modern method for extracting the components of interest from the natural raw material efficiently is a carbon dioxide (CO₂) extraction, which uses carbon dioxide in the form of fluid or supercritical fluid for extracting.

The sea buckthorn seed oil has typically a distinct colour that varies from orange to yellow. The colour of the seed oil may cause unwanted pigmentation and contamination problems in some applications. Due to these problems, the use of the sea buckthorn seed oil has been restricted.

### OBJECTIVES OF THE INVENTION

It is the main objective of the present invention to reduce or even eliminate the problems in the prior art.

It is an object of the present invention to provide a seed oil which is safe to use, provides health benefits and can be freely used in various kinds of applications.

Another object of the present invention is to provide a seed oil which is rich in selected bioactive components and suitable for manufacture of various cosmetic products and medical devices without extensive purification steps.

Still another object of the present invention is to provide a composition which has an antioxidant activity.

### DESCRIPTION OF THE INVENTION

A seed oil according to the invention which is obtainable from seeds of *Hippophae rhamnoides* by supercritical CO₂ extraction comprises
- at least 25 weight-% linoleic acid, calculated from the total fatty acids in the seed oil,
- at least 20 weight-% alpha-linolenic acid, calculated from the total fatty acids in the seed oil,
- less than 0.001 weight-% carotenoids, based on the total weight of the seed oil, and
wherein the amount of polycyclic aromatic hydrocarbons (PAHs), selected from the group consisting of benzo(a)pyrene, chrysene, benz(a)anthracene and benzo(a)fluoranthene is less than 10 µg/kg seed oil.

A composition according to invention comprises
- the seed oil according to present invention, and
- an antioxidant agent, such as rosemary extract and/or alpha-tocopherol in an oil vehicle.

Now it has been surprisingly found out that when the total carotenoid content of the seed oil obtained from the seeds of *Hippophae rhamnoides* by supercritical CO₂ extraction is less than 0.001 weight-%, the oil is practically colourless. This means that the use of the seed oil according to invention is not restricted due to the colour. The low content of carotenoids and colourlessness of the seed oil enables the seed oil to be freely used in the topical applications and in places where the seed oil could otherwise cause unwanted colouring, for example, in face, visible parts of the skin, intimate area, and eye applications, such as eye drops and eye cream. When also the amount of polycyclic aromatic hydrocarbons (PAHs), which are selected from the group consisting of benzo(a)pyrene, chrysene, benz(a)anthracene and benzo(a)fluoranthene, is less than 10 µg/kg seed oil, the seed oil can be safely used in applications for the mucous membranes and in other sensitive applications, for example, for eyes.

It has also been found out that with specific amount of essential fatty acids and especially with antioxidant agent, the seed oil according to invention is especially suitable, for example, for moisturising and taking care of mucous membranes and skin.

The berries of *Hippophae rhamnoides,* commonly referred to as sea buckthorn, are used as a raw material for the seed oil production. *Hippophae rhamnoides* is the most widespread of the species in the genus *Hippophae* and has several subspecies, such as *Hippophae rhamnoides* ssp. *sinensis,* ssp. *rhamnoides* and ssp. *mongolica*. Berry seeds of different subspecies comprise different chemical compounds in different amounts. The chemical composition of the seed oil may be controlled and tailored by careful selection of the used raw material. The seeds of all subspecies of *Hippophae rhamnoides,* such as *Hippophae rhamnoides* ssp. *sinensis,* ssp. *rhamnoides* or ssp. *mongolica* may be used as a raw material for the seed oil production. The seeds of *Hippophae rhamnoides ssp. sinensis* are preferred as a raw material.

Typically, the seeds of *Hippophae rhamnoides* contain carotenoids, which are pigments that have given oil its distinctive colour. The total content of carotenoids in the seeds varies greatly between different growth locations and subspecies. Typical carotenoids in the seeds are beta-carotene, lycopene, lutein and zeaxanthin. The amount of carotenoids can be reduced from the seed oil, for example, by bleaching. The bleaching can be done by physical adsorption of carotenoids using adsorbent agents.

The seed oil according to the present invention may comprise less than 0.001 weight-%, preferably less than 0.0007 weight-%, more preferably less than 0.0006 weight-% carotenoids, based on the total weight of the seed oil. According to one embodiment of the invention, the seed oil comprises 0.0001-0.001 weight-%, preferably 0.0003-0.0007 weight-%, more preferably 0.0004-0.0006 weight-% carotenoids, based on the total weight of the seed oil. The amount of carotenoids in the seed oil is measured with UV-Spectrophotometer (Shimadzu UV 1700) in hexane at 450 nm. Because the seed oil according to invention is practically free of carotenoids and practically colourless, the use of the seed oil is not restricted. It can be freely used in various applications, such as skin and eye formulations. Also the amount of the seed oil can be increased in products where the colourless composition is needed.

Especially, in the formulations for use in eye area, the low amount or absence of carotenoids is preferred in order to be able to use products with the seed oil with contact lenses. Otherwise, the carotenoids could stick to the contact lenses and colour them.

Furthermore, a high amount of carotenoids may cause carotenermia which is a clinical condition characterized by yellow pigmentation of the skin and increased beta-carotene levels in the blood. The seed oil according to the invention, which comprises less than 0.001 weight-% of carotenoids, suits as a source of other active agents, for example fatty acids, for eatable products which itself contains high amounts of carotenoids or for products which are used in high doses. Beta-carotene may also pose a health risk for smokers. Thus, the seed oil according to the invention can be used in the products used by smokers.

The seeds of *Hippophae rhamnoides* may also contain polycyclic aromatic hydrocarbons (PAHs), which are harmful chemical contaminants having toxic, mutagenic and/or carcinogenic properties. The amount of these contaminants in the seed oil is preferably at low level. According to the invention, the amount of polycyclic aromatic hydrocarbons (PAHs), selected from the group consisting of benzo(a)pyrene, chrysene, benz(a)anthracene and benzo(a)fluoranthene is less than 10 µg/kg seed oil, preferably less than 8 µg/kg seed oil, more preferably less than 6 µg/kg seed oil. Preferably, the sum of four marker PAHs benzo(a)pyrene, chrysene, benz(a)anthracene and benzo(a)fluoranthene (ΣPAH4) is less than 10 µg/kg seed oil. The sum of the marker PAHs can be used as an indicator of the total concentration of all PAHs. The amount of PAHs in the seed oil is measured with gas chromatograph with mass spectrometry detector (GC-MS). Due to the low amount of the contaminants, the seed oil can be used in applications for the mucous membranes and in other sensitive applications, for example, for eyes. When the amount of PAHs in the seed oil is at the low level, the use of the seed oil according to invention is not restricted due to the harmful contaminant. The low amount of both, the carotenoids and the PAHs, in the seed oil enables the seed oil to be especially suitable for and safe to use in sensitive applications and in visible areas, such as eye applications like eye drops and eye cream.

The oil according to the present invention is extracted from the seeds of *Hippophae rhamnoides* berries by supercritical CO₂ extraction where carbon dioxide is used to extract the oil out while retaining the beneficial components in active form. Among the different oil manufacturing technologies, supercritical CO₂ extraction provides a good quality due to the high extraction efficiency and the absence of harmful solvent residues in the oil extracted. Due to the absence of harmful solvent residues, supercritical CO₂ -extracted seed oils are safe to use as ingredient for cosmetics, health and personal care products and medical devices. The reduction of carotenoids and PAHs from the seed oil is done preferably during the supercritical CO₂ extraction. This may remove the requirement of additional purification steps after the extraction of the seed oil and speeds up the production.

The seed oil of *Hippophae rhamnoides* is rich in essential fatty acids, which cannot be synthesized in the body and must be obtained from the food. Essential fatty acids are important in the normal functioning of all tissues of the body. Total fatty acid content in the seed oil according to the invention may be at least 92 weight-%, based on the total weight of the seed oil. The seed oil according to the present invention is rich in linoleic acid (C18:2, omega-6), the amount of linoleic acid being at least 25 weight-%, preferably at least 27 weight-%, more preferably at least 30 weight-% calculated from the total fatty acids in the seed oil. The amount of linoleic acid in the seed oil may be less than 60 weight-%, preferably less than 50 weight-%, more preferably less than 45 weight-% calculated from the total fatty acids in the seed oil. The seed oil may comprise 25-60 weight-%, preferably 27-50 weight-%, more preferably 30-45 weight-% linoleic acid calculated from the total fatty acids in the seed oil. The amount of linoleic acid in the seed oil is measured with gas chromatograph-flame ionization detector (GC-FID) as methyl esters.

General structure of linoleic acid is shown below:

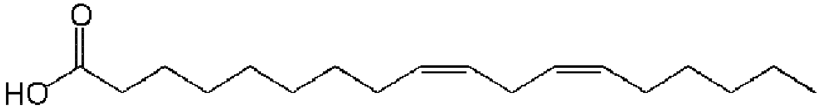

The seed oil according to the present invention is also rich in alpha-linolenic acid (C18:3, omega-3), the amount of alpha-linolenic acid being at least 20 weight-%, preferably at least 22 weight-%, more preferably at least 24 weight-% calculated from the total fatty acids in the seed oil. The amount of alpha-linolenic acid in the seed oil may be less than 50 weight-%, preferably less than 45 weight-%, more preferably less than 38 weight-% calculated from the total fatty acids in the seed oil. The seed oil may comprise 20-50 weight-%, preferably 22-45 weight-%, more preferably 24-38 weight-% alpha-linolenic acid calculated from the total fatty acids in the seed oil. The amount of alpha-linolenic acid in the seed oil is measured with gas chromatograph-flame ionization detector (GC-FID) as methyl esters.

General structure of alpha-linolenic acid is shown below:

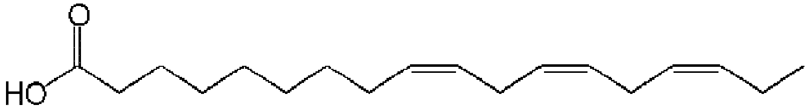

The seed oil of *Hippophae rhamnoides* may also comprise oleic acid (C18:1, omega-9), which is monounsaturated fatty acid occurring naturally in various animal and vegetable fats and oils. Oils containing oleic acid are great for softening skin, regenerating skin cells, moisturizing, and behaving as an antiinflammatory. The seed oil may comprise at least 9 weight-%, preferably at least 10 weight-%, more preferably at least 11 weight-% oleic acid calculated from the total fatty acids in the seed oil. The amount of oleic acid in the seed oil may be less than 35 weight-%, preferably less than 30 weight-%, more preferably less than 25 weight-% calculated from the total fatty acids in the seed oil. The seed oil may comprise 9-35 weight-%, preferably 10-30 weight-%, more preferably 11-25 weight-% oleic acid calculated from the total fatty acids in the seed oil. The amount of oleic acid in the seed oil is measured with gas chromatograph-flame ionization detector (GC-FID) as methyl esters.

General structure of oleic acid is shown below:

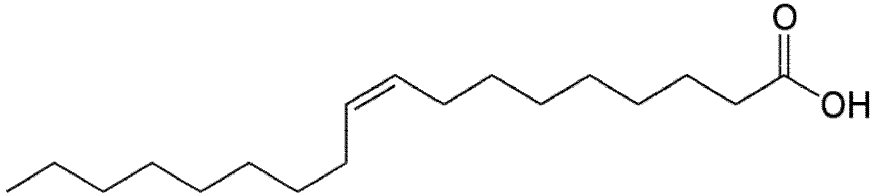

The amount of solid and/or chemical contaminants in the seed oil is preferably at low level. According to one embodiment of the invention, the seed oil comprises solid material less than 2 weight-% of the total weight of the seed oil. Possible disturbing solid material may be separated from the obtained seed oil for example by filtration and/or centrifugation. According to another embodiment of the invention, the seed oil extracted by supercritical CO₂ extraction is free of solid matter and organic solvents.

Also the peroxide value which describes the oxidation of the seed oil and/or acid value which describes the hydrolysis of the seed oil are preferably low. According to one embodiment of the invention the peroxide value of the seed oil is less than 15 meq/kg seed oil, preferably less than 13 meq/kg seed oil, more preferably less than 10 meq/kg seed oil and/or the acid value of the seed oil is less than 18 mg KOH/g seed oil, preferably less than 14 mg KOH/g seed oil, more preferably less than 10 mg KOH/g seed oil. The peroxide value and the acid value of the seed oil are measured with titration methods. Due to the low level of oxidation and/or hydrolysis of the seed oil, it can be used in sensitive applications, for example, for eyes and the mucous membranes.

According to one embodiment of the invention, the seed oil is essentially free of microbes, yeasts and moulds. In this context essentially free denotes that the total aerobic microbial count (TAMC) of the seed oil is less than 10 cfu/g seed oil, measured using the method in accordance with the European Pharmacopoeia, and/or the total combined yeasts/moulds count (TYMC) of the seed oil is less than 10 cfu/g seed oil, measured using the method in accordance with the European Pharmacopoeia. This ensures the applicability of the seed oil for sensitive applications and improves shelf life of the seed oil.

According to one embodiment of the invention, the amount of following allergens: 2-benzylideneheptanal, amylcinnamyl alcohol, benzylalcohol, benzyl salicylate, cinnamylic alcohol, cinnamaldehyde, citral, coumarin, eugenol, geraniol, 7-hydroxycitronellal-lyral, isoeugenol, 4-methoxybenzylic alcohol, benzyl benzoate, benzyl cinnamate, citronellol, farnesol, hexylcinnamaldehyde, lilial, limonen, linalool, methyl oct-2-ynoate and alpha-ketone is less than 10 mg/kg seed oil (Directive 2003/15/EC of the European Parliament and of the Council). According to another embodiment of the invention, the seed oil is free of common compounds that cause contact-allergy reactions.

According to one embodiment of the invention the seed oil further comprises at least one tocopherol, such as alpha- and/or gamma-tocopherol, and/or at least one phytosterol, such as sitosterol. Alpha-tocopherol is the major vitamin E compound in sea buckthorn seed oil. Alpha-tocopherol is a form of vitamin E that is preferentially absorbed and accumulated in humans. Seed oil also contains considerable amounts of gamma-tocopherol, which is also one of the chemical compounds that is considered vitamin E. The high vitamin E equivalency provides protection against oxidation and makes the seed oil useful natural antioxidant. Phytosterols are plant sterols with structures related to cholesterol. Phytosterols are known to have cholesterol reducing properties. Sitosterol (beta-sitosterol) is the major phytosterol in sea buckthorn seed oil. Sitosterol behaves like cortison, offering a reduction in redness and inflammation, and soothing of itchy skin.

According to one embodiment of the invention the total amount of the tocopherols and/or phytosterols is less than 3 weight-%, preferably less than 2.5 weight-%, more preferably less than 2 weight-%, based on the total weight of the seed oil. The seed oil may comprise in total 0.1-3 weight-%, preferably 0.5-2.5 weight-%, more preferably 1-2 weight-% tocopherols and/or phytosterols based on the total weight of the seed oil. The amount of tocopherols and phytosterols is measured with high-performance liquid chromatograph-UV detector (HPLC-UV) and with gas chromatograph-flame ionization detector GC-FID.

A composition according to the present invention comprises the seed oil according to the invention and an antioxidant agent, such as rosemary extract and/or alpha-tocopherol in an oil vehicle. Rosemary extract is derived from *Rosmarinus officinalis* L. and contains several compounds which have been proven to exert antioxidative functions. These compounds belong mainly to the classes of phenolic acids, flavonoids, diterpenoids and triterpenes. Also alpha-tocopherol can be added to the composition to improve shelf life of the composition.

According to one embodiment of the invention, the amount of the antioxidant agent in the composition is 2.1-4 weight-%, preferably 1.1-2 weight-%, more preferably 0.05-1 weight-% of the total weight of the composition.

According to one embodiment of the invention, the composition comprises at least 0.1 weight-%, preferably at least 0.2 weight-%, more preferably at least 0.4 weight-% seed oil of the total weight of the composition. The composition may comprise less than 50 weight-%, preferably less than 25 weight-%, more preferably less than 10 weight-% seed oil of the total weight of the composition. The amount of the seed oil in the composition may also be 0.1-50 weight-%, preferably 0.2-25 weight-%, more preferably 0.4-10 weight-% of the total weight of the composition. The composition may also comprise other additional ingredients, such as additives and moisture binding compounds.

The seed oil that is obtainable by extracting the seeds of *Hippophae rhamnoides* by supercritical CO₂ extraction can be used as an ingredient for health care, cosmetic, personal care products or animal care products. It may also be used for manufacture of functional foods, food supplements and animal foods.

The seed oil according to the invention may be used as skin nourishing component and natural antioxidant as well as an ingredient of peeling and scrubbing composition. The nourishing and conditioning effects of the seed oil are based on the fatty acids. Examples of the products, where the seed oil may be used, are orally administered products for skin care, topical creams and lotions, face scrubs, face gels, shower gels, shampoos and hair conditioners.

Cosmetics, such as face creams or body lotions may comprise at least 0.5 weight-%, preferably at least 0.7 weight-%, more preferably at least 1 weight-% seed oil of the total weight of the end product. Cosmetics may comprise less than 7 weight-%, preferably less than 6 weight-%, more preferably less than 5 weight-% seed oil of the total weight of the end product. The seed oil may be used in cosmetics, such as face creams or body lotions, in amount corresponding to 0.5-7 weight-%, preferably 0.7-6 weight-%, more preferably 1-5 weight-% of the total weight of the end the product.

The seed oil according to the invention may be used in treatment of eye, nose, mouth, intimate area, gastric, urogenital or skin and scalp disorders. The treatment may be in the form of spray, lotion, drops or orally administered product.

The seed oil may be used in a treatment in amount corresponding to at least 0.01 weight-%, preferably at least 0.1 weight-%, more preferably at least 0.3 weight-% of the total weight of the end product. The seed oil may also be used in a treatment in amount corresponding to less than 50 weight-%, preferably less than 35 weight-%, more preferably less than 20 weight-% of the total weight of the end product. According to another embodiment of the invention the seed oil is used in a treatment as hydrating, repairing and relieving symptoms and signs of dryness, irritation and inflammation, in amount corresponding to 0.01-50 weight-%, preferably 0.1-35 weight-%, more preferably 0.3-20 weight-% of the total weight of the end product. It may also be used for maintaining skin health and reducing skin ageing, for maintaining and improving skin elasticity, or as functional ingredients for promoting skin regeneration and wound healing.

The invention is more closely described in the following non-limiting examples.

### EXPERIMENTAL PART

### Example 1

Genetic background and growth conditions of the *Hippophae rhamnoides* affects to the composition of the seeds. By careful selection of the seeds, the chemical compounds in the seed oil may be tailored after requirements. The berries of *Hippophae rhamnoides* delivered from Asia are preferred as a raw material. The berries are collected from the shrubs which typically grow at altitude of 800 m above sea level and at latitude of 40-55 degrees north. The berries are first dried and the seeds are separated from the raw material, after which the composition of the seeds is analysed. If the composition is as wanted, the seeds are ground before extraction.

### Example 2

The seeds of *Hippophae rhamnoides* are analysed after separating from the raw material berries. The total oil content in the seeds is typically at least 8 weight-% and the moisture content less than 10 weight-% of the total dry weight of the seeds.

It may be concluded that these seeds may be used as promising starting material for extraction.

### Example 3

The extraction is carried out by using supercritical CO₂. By adjusting pressure and temperature of the extraction process, the wanted composition of the seed oil is obtained. Colours are eliminated from the composition by reduction of natural compounds that produces colours (carotenoid) by bleaching. The bleaching and the reduction of PAHs are performed during the supercritical CO₂ extraction. The seed oil obtained from CO₂ extraction is analysed. Table 1 shows amounts of selected compounds in the seed oil and the analysis methods of them.

**Table 1. Composition of CO₂ extracted seed oil of Hippophae rhamnoides berries.**

| **Name** | **Method** | **Value** | **Unit** |
|---|---|---|---|
| C18:1 (n-9) | GC-FID | 20.7 | % |
| C18:2 (n-6) | GC-FID | 39 | % |
| C18:3 (n-3) | GC-FID | 27.4 | % |
| Carotenoids | UV-Spectrophotometer | <0.0003 | % |
| Tocopherols | HPLC-UV | 0.09 | % |
| Peroxide value | Titration methods* | 5.1 | meq/kg |
| Acid value | Titration methods* | 4.5 | mg KOH/g |
| ΣPAH4 (benzo(a)pyrene, chrysene, benz(a)anthracene and benzo(a)fluoranthene) | GC-MS | <2 | µg/kg |
| Density | Pycnometer | 0.92 | g/cm³ |
| TAMC (Total aerobic microbial count) | Ph. Eur.** | <10 | cfu/g |
| TYMC (Total combined yeasts/moulds count) | Ph. Eur** | <10 | cfu/g |

| | | | |
|---|---|---|---|
| * standard DGK Meth. no. W 010.1 Ausgabe 1/91 DGF Unit Method G-III 13, standard SFS-EN ISO 660. ** measured using the method in accordance with the European Pharmacopoeia. | | | |

The amount of contaminants in the composition is minimised, so, the oil can be used in the applications of the mucous membranes and in other sensitive applications. The seed oil preferably does not contain common compounds that cause contact-allergy reactions. The amount of following allergens 2-benzylideneheptanal, amylcinnamyl alcohol, benzylalcohol, benzyl salicylate, cinnamylic alcohol, cinnamaldehyde, citral, coumarin, eugenol, geraniol, 7-hydroxycitronellal-lyral, isoeugenol, 4-methoxybenzylic alcohol, benzyl benzoate, benzyl cinnamate, citronellol, farnesol, hexylcinnamaldehyde, lilial, limonen, linalool, methyl oct-2-ynoate, alpha-ketone in accordance with Directive 2003/15/EC of the European Parliament and of the Council are analysed to be less than 10 mg/kg of the seed oil.

As a secondary treatment after the extraction, a rosemary extract is added to the seed oil. An intermediate product of the seed oil and rosemary extract is packed to the protective gas. Waxes which cause opacity to the composition are removed and the final composition is packed again to the protective gas.

The seed oil obtained from CO₂ extraction may be used as food, food supplements, nutraceuticals, medical devices, cosmetics, health and personal care products as well as ingredients in these product groups. The concentration level of the seed oil in the final products may vary from 0.01 weight-% to 98 weight-% of the total weight of the final product.

## Claims

1. A seed oil, obtainable from seeds of *Hippophae rhamnoides* by supercritical CO₂ extraction, the seed oil comprising:
- at least 25 weight-% linoleic acid, calculated from the total fatty acids in the seed oil,
- at least 20 weight-% alpha-linolenic acid, calculated from the total fatty acids in the seed oil,
- less than 0.001 weight-% carotenoids, based on the total weight of the seed oil, and
wherein the amount of polycyclic aromatic hydrocarbons (PAHs), selected from the group consisting of benzo(a)pyrene, chrysene, benz(a)anthracene and benzo(a)fluoranthene is less than 10 µg/kg seed oil.

2. Seed oil according to claim 1, **characterised in that** the seed oil comprises 25-60 weight-%, preferably 27-50 weight-%, more preferably 30-45 weight-% linoleic acid, calculated from the total fatty acids in the seed oil.

3. Seed oil according to claim 1 or 2, **characterised in that** the seed oil comprises 20-50 weight-%, preferably 22-45 weight-%, more preferably 24-38 weight-% alpha-linolenic acid, calculated from the total fatty acids in the seed oil.

4. Seed oil according to claim 1, 2 or 3, **characterised in that** the seed oil comprises 0.0001-0.001 weight-%, preferably 0.0003-0.0007 weight-%, more preferably 0.0004-0.0006 weight-% carotenoids, based on the total weight of the seed oil.

5. Seed oil according to claim 1 to 4, **characterised in that** the seed oil comprises at least 9 weight-% oleic acid, calculated from the total fatty acids in the seed oil.

6. Seed oil according to claim 1 to 5, **characterised in that** the seed oil comprises 9-35 weight-%, preferably 10-30 weight-%, more preferably 11-25 weight-% oleic acid, calculated from the total fatty acids in the seed oil.

7. Seed oil according to any of the preceding claims 1-6, **characterised in that** the peroxide value of the seed oil is less than 15 meq/kg seed oil and/or acid value of the seed oil less than 18 mg KOH/g seed oil.

8. Seed oil according to any of the preceding claims 1-7, **characterised in that** the seed oil further comprises at least one tocopherol, such as alpha- and gamma-tocopherol, and/or at least one phytosterol, such as sitosterol.

9. Seed oil according to claim 8, **characterised in that** the total amount of the tocopherols and/or phytosterols is typically less than 3 weight-%, based on the total weight of the seed oil.

10. A composition, comprising:
- the seed oil according to any of claims 1-9, and
- an antioxidant agent, such as rosemary extract and/or alpha-tocopherol in an oil vehicle.

11. Composition according to claim 10, **characterised in that** the amount of the seed oil is 0.1-50 weight-%, preferably 0.2-25 weight-%, more preferably 0.4-10 weight-% of the total weight of the composition.

12. Composition according to claim 10 or 11, **characterised in that** the amount of the antioxidant agent is 2.1-4 weight-%, preferably 1.1-2 weight-%, more preferably 0.05-1 weight-% of the total weight of the composition.

13. The seed oil according to any of the preceding claims 1-9 for use in treatment of eye, nose, mouth, intimate area, gastric, urogenital or skin and scalp disorders.

14. The seed oil for use according to claim 13, **characterised in that** the seed oil is used in a treatment as hydrating and repairing, relieving symptoms and signs of dryness, irritation and inflammation, in amount corresponding to 0.01-50 weight-%, preferably 0.1-35 weight-%, more preferably 0.3-20 weight-% of the total weight of the end product.
